# FASCICULE DE BREVET EUROPEEN

(11) **EP 1 715 813 B1**
(45) Date de publication et mention de la délivrance du brevet: **29.05.2013**
(21) Numéro de dépôt: 05717469.0
(22) Date de dépôt: 21.01.2005
(51) Int. Cl.: A61F 2/08

(54) **PROCEDE POUR LA FIXATION DE FILS DE TRACTION AUX EXTREMITES D'UN LIGAMENT PROTHETIQUE**
VERFAHREN ZUR BEFESTIGUNG VON TRAKTIONSFÄDEN AN DEN ENDEN EINER LIGAMENTPROTHESE
METHOD FOR FIXING TRACTION THREADS TO ENDS OF A PROSTHETIC LIGAMENT

(30) Priorité: 23.01.2004 FR 0400630
(43) Date de publication de la demande: 02.11.2006
(73) Titulaire: L.A.R.S. - laboratoire d'application et de Recherche Scientifique, 21560 Arc-sur-Tille (FR)
(72) Inventeur: BRULEZ, Bernard, F-52400 Bourdonne-les-Bains (FR); LABOUREAU, Jacques-Philippe, F-06530 Le Tignet (FR)
(74) Mandataire: Puiroux, Guy
(86) Numéro de dépôt international: PCT/FR2005/000142
(87) Numéro de publication internationale: WO 2005/079707

(56) Documents cités:
- WO-A-89/01320
- FR-A- 2 710 520
- FR-A- 2 729 559
- FR-A- 2 755 846
- US-A- 4 187 558
- US-B1- 6 203 572

## Description

La présente invention concerne un procédé pour la fixation de fils de traction aux extrémités d'un ligament prothétique pour le remplacement de ligaments articulaires biologiques, et notamment ceux du genou, et le ligament ainsi obtenu.

La plupart des ligaments artificiels sont aujourd'hui fabriqués par roulage ou pliage sur elle-même d'une laize en fibres polymères synthétique, généralement du polyéthylène téréphtalate. Ces ligaments ont la forme globale de cylindres allongés comprenant entre deux parties extrêmes intra-osseuses une partie médiane intra-articulaire. Des exemples de tels ligaments sont notamment donnés dans les brevets FR 2.755.846 et FR 2.697.151. Le document US 6.203.572 décrit un procédé de confection d'un ligament prothétique selon le préambule de la revendication 1.

Afin de faciliter le passage de ces ligaments dans les tunnels osseux et d'assurer leur bonne mise en place, les ligaments sont munis à leurs extrémités de fils de traction. Ces fils de traction sont montés sur les ligaments en effectuant à une certaine distance de leurs extrémités libres, une boucle avec un fil enserrant radialement le ligament ; les deux brins de ce fil sont introduits dans l'épaisseur du ligament et ressortent par l'extrémité libre du ligament à laquelle on adjoint ledit fil, après avoir tiré les deux brins dans l'épaisseur du ligament selon une direction globalement parallèle à l'axe longitudinal du ligament. Puis on noue entre eux les deux brins, au niveau de leur point de sortie du ligament.

Pour protéger les extrémités libres du ligament, guider son passage dans les tunnels osseux et éviter tout déplacement des fils de traction lorsqu'ils sont soumis à un effort de traction, diverses solutions d'embout ont été élaborées par les fabricants de ligaments. Dans le présent texte, le terme « embout » désigne toute pièce venant coiffer les extrémités munies des fils de traction d'un ligament prothétique.

Une première de ces solutions consiste à enserrer le ligament au dessous de la boucle du fil de traction avec une bague, habituellement métallique. Or ces bagues se bloquent souvent dans les tunnels osseux et passent difficilement les coudes, rendant la pose de ligaments peu aisée et parfois délicate.

Une deuxième solution est de poser sur l'extrémité du ligament un capuchon en polyéthylène, muni d'un trou en son fond par lequel passent les brins du fil de traction. On noue entre eux les deux brins du fil de traction juste à leur sortie du capuchon, pour immobiliser ce dernier et empêcher le fil de glisser. Si ces capuchons sont moins problématiques que les bagues, ils restent d'utilisation difficile dans certains cas de figure, par exemple dans le cas du remplacement de ligaments croisés postérieurs du genou. En outre, ces capuchons sont parfois associés à une bague, augmentant les risques inflammatoires du fait de la nature différente des matériaux employés.

Une troisième solution utilisée par certains fabricants consiste en des gaines de film polymère plastique (PVC) enserrant l'extrémité du ligament qui sont ouvertes au point de sortie des brins du fil de traction. Ces gaines peuvent être obtenues par thermoformage, par exemple. Elles ont comme inconvénient d'être longues et relativement rigides, rendant difficile le passage du ligament dans les tunnels osseux.

Enfin, une autre solution met- en oeuvre des guides en silicone, moulés autour du fil de traction et présentant une forme de cône allongé. Si ces guides ont la souplesse requise, ils perdent de la matière dans les tunnels osseux et provoquent des bourrages, du fait de leur taille importante. De plus, les particules émises présentent un risque de réaction inflammatoire.

La présente invention vise donc à remédier à ces problèmes en proposant un procédé de confection d'un ligament prothétique, comportant une étape de roulage ou pliage sur elle-même d'une laize de fibres polymères synthétiques, suivie d'une étape de pose d'un fil de traction à chacune des extrémités du ligament, puis d'une étape de mise en place d'un embout sur les extrémités, remarquable en ce que l'étape de mise en place de l'embout consiste à réaliser une ligature radiale du ligament muni de ses fils de traction avec un fil pour ligature.

L'expression « fil pour ligature » désigne dans le présent texte un fil textile dont les caractéristiques techniques le destinent à une utilisation habituelle pour la réalisation de ligatures.

On comprend bien qu'un tel embout consistant en une ligature est souple et ne crée aucune surépaisseur gênante sur l'extrémité du ligament ; il supprime ainsi les difficultés de pose du ligament, notamment lors du passage dans les tunnels osseux et plus spécialement dans les coudes, quelle que soit leur conformation.

En outre, selon une caractéristique essentielle de l'invention, le fil pour ligature est choisi dans le même matériau que celui des fibres synthétiques constituant la laize. Les risques de réaction inflammatoire chez le patient sont donc considérablement réduits puisque l'embout est de même nature que le matériau constituant le ligament et qu'en plus le rejet de particules de matière est extrêmement faible, voire inexistant. On notera également l'avantage économique certain à utiliser une même matière première.

Enfin, on connaît bien la résistance et la solidité des ligatures et autres noeuds lorsqu'ils sont correctement effectués.

D'autres avantages et caractéristiques ressortiront mieux de la description qui va suivre d'une succession des étapes du procédé, donnée à titre d'exemple non limitatif, pour le montage des fils de traction sur un ligament prothétique, après roulage ou pliage sur elle-même d'une laize en fibres polymères, en référence aux figures annexées sur lesquelles:
- la figure 1 est une vue schématique en perspective d'un ligament artificiel.
- la figure 2 représente la séquence d'une variante selon l'invention de la pose du fil de traction à l'extrémité d'un ligament;
- la figure 3 représente la séquence d'un mode de réalisation de la ligature constituant un embout souple selon l'invention.

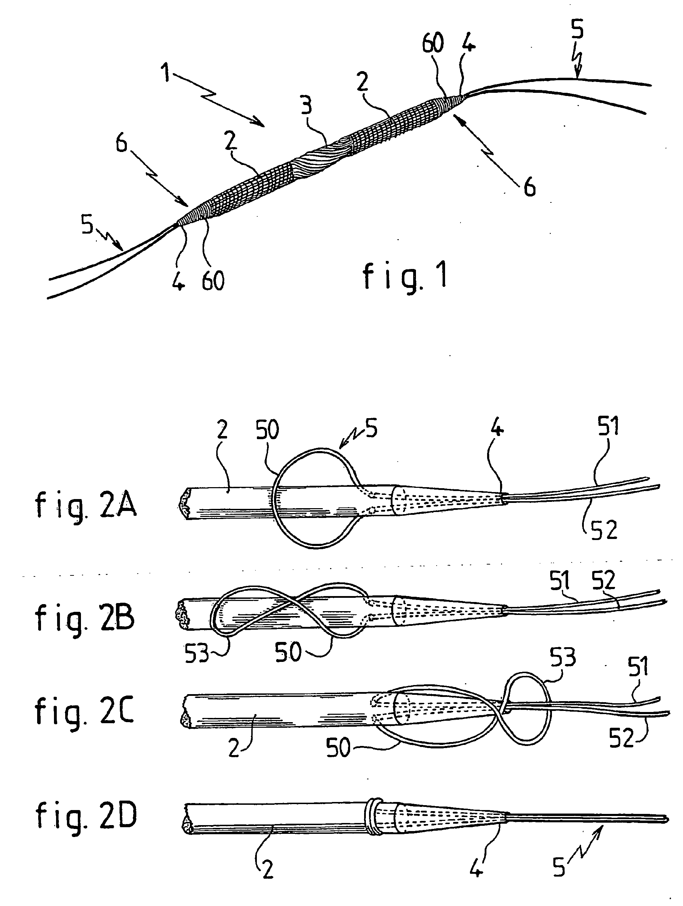

En référence à la figure 1, un ligament artificiel 1 a la forme globale d'un cylindre allongé comprenant entre deux parties extrêmes intra-osseuses 2 une partie médiane intra-articulaire 3. Le ligament est muni à chacune de ses extrémités 4 de fils de traction 5, les extrémités étant recouvertes par un embout 6, qui est ici une ligature 60, réalisée selon le procédé détaillé ci-après. Ces ligaments artificiels sont obtenus par roulage ou pliage sur elle-même d'une laize en fibres polymères synthétiques, qui sont généralement du polyéthylène téréphtalate. Cette laize est découpée et profilée afin de conformer les extrémités 4 du ligament 1 à la forme voulue, puis roulée. On trouvera des exemples de ligaments de ce type détaillés avec leur procédé d'obtention dans les brevets FR 2.755.846 ou FR 2.697.151, par exemple. Enfin, la forme ainsi donnée au ligament 1 est figée par une couture longitudinale du ligament.

On procède ensuite à la mise en place des fils de traction 5, nécessaires à la pose du ligament chez les patients, pour permettre au praticien d'assurer le passage du ligament dans les tunnels osseux ainsi que son positionnement correct et la tension requise pour la réparation.

En référence à la figure 2, et selon une caractéristique de l'invention, la mise en place du fil de traction 5 à l'extrémité du ligament s'effectue selon une séquence d'étapes illustrées par les figures 2A à 2D. La première étape de la séquence consiste à prendre un fil de traction 5, puis à former une boucle 50 autour du corps du ligament 1, sans croiser les brins autour de ce dernier, et à disposer ladite boucle 50 à une certaine distance de l'extrémité 4 du ligament 1. Cette distance varie généralement de 20 à 50 mm de l'extrémité, selon le type de ligament considéré. Par exemple, dans le cas d'un ligament dont les extrémités ont une forme conique, cette distance varie généralement entre 30 et 40 mm.

On prend ensuite les deux brins libres 51,52 du fil de traction et on les introduit en opposition diamétrale de la boucle 50 dans l'épaisseur du ligament 1. Puis on les tire à l'extérieur du ligament, vers l'extrémité 4 considérée, parallèlement à la direction longitudinale du ligament. On veillera lors de cette étape à ne pas réduire la taille de la boucle 50.

On imprime ensuite, comme illustré sur la figure 2B, une vrille à la boucle 50 de façon à former un huit dont la base est ancrée dans le ligament et qui forme une deuxième boucle 53 correspondant à la petite partie du huit. Puis on introduit l'extrémité 4 du ligament avec les brins libres 51,52 à l'intérieur de la deuxième boucle 53 et on ramène la première et la deuxième boucle reliées en huit à la hauteur du point d'introduction des deux brins libres de fils dans le ligament. Enfin, on serre le noeud ainsi formé en tirant sur les brins 51,52 du fil de traction 5.

De manière optionnelle et avantageuse, on réalise ensuite un point d'arrêt sur l'extrémité 4 du ligament, au niveau de la section du ligament dans laquelle les deux brins 51,52 passent dans l'épaisseur du ligament. Ce point d'arrêt est destiné à empêcher tout glissement du fil de traction 5 lorsque celui-ci est soumis à une forte traction, notamment lors de la pose du ligament. Le point d'arrêt est exécuté par exemple avec une machine à coudre comportant une programmation point d'arrêt. Bien entendu, il est possible de procéder différemment pour la mise en place du fil de traction, mais la méthode qui vient d'être décrite ici a l'avantage d'être extrêmement simple, rapide et de ne pas nécessiter de noeuds supplémentaires après la pose de l'embout protégeant l'extrémité du ligament.

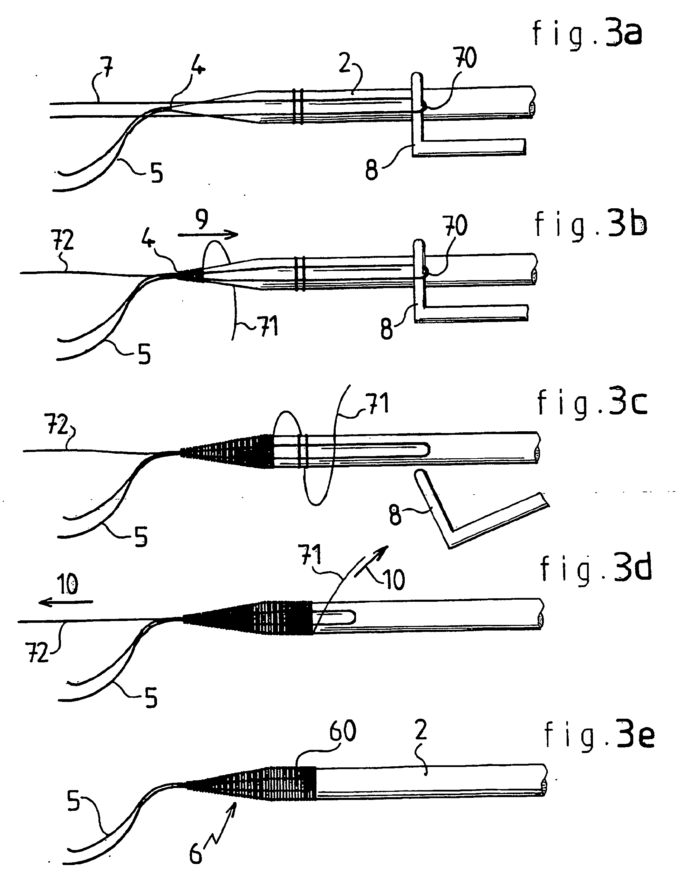

En référence à la figure 3 et selon une autre caractéristique importante de l'invention, l'étape suivante de mise en place d'un embout 6 sur les extrémités 4 du ligament 1 muni de ses fils de traction 5 consiste à réaliser une ligature radiale 60 dudit ligament avec un fil 7 pour ligature.

Cette ligature 60 est réalisée selon la séquence illustrée par les figures 3A à 3E.

La première étape de réalisation de la ligature 60 consiste à former une boucle 70 allongée, à partir d'un fil 7 pour ligature tiré d'une bobine de fil, et à disposer ladite boucle 70 sur le ligament 1, les deux brins du fil de ligature étant parallèles au ligament et débouchant à l'extrémité 4 du ligament sur laquelle on met en place ladite ligature 60. Le ligament 1 est posé à plat, de préférence maintenu à l'aide d'un dispositif approprié et la boucle 70 est avantageusement maintenue contre le ligament par un crochet amovible 8. Ce crochet amovible 8 fait partie du précédent dispositif de maintien à plat du ligament 1. Il permet avantageusement de libérer l'opérateur devant réaliser la ligature 60 de la contrainte de maintenir avec un doigt, par exemple, la boucle 70 contre le ligament 1 pendant l'exécution des étapes suivantes de réalisation de ligature.

On prend ensuite l'un des brins du fil 7, qui devient le brin courant 71, et on l'enroule simultanément autour du ligament, autour du second brin 72, appelé dormant, s'étendant toujours parallèlement à la direction longitudinale du ligament 1, et autour de la boucle 70, en partant de l'extrémité 4 du ligament et en remontant en direction de la partie médiane intra-articulaire 3, selon la direction indiquée par la flèche 9 (fig. 3B). On veillera en enroulant le brin courant 71 à former des spires bien jointives.

On passe ensuite le brin courant 71 dans la boucle 70 et on dégage celle-ci du crochet 8 qui la maintenait en place, comme illustré à la figure 3C. Généralement, on effectue cette étape lorsque les spires formées ont recouvert le double noeud du fil de traction 5, à la hauteur du point d'insertion des brins 51,52 dudit fil 5 dans l'épaisseur du ligament 1. Bien entendu, on pourra réduire ou augmenter la surface recouverte par les spires, et de fait, la taille de la ligature 60, en fonction des besoins spécifiques à chaque type de ligament prothétique, sans pour autant sortir du cadre de l'invention.

Puis, on serre fortement en tirant sur le brin dormant 72 et le brin courant 71 dans les directions représentées par les flèches 10 sur la figure 3D, respectivement dans l'axe longitudinal du ligament pour le brin dormant 72 et globalement perpendiculaire à celui-ci pour le brin courant 71, tout en maintenant les spires bien jointives.

Enfin, on coupe les deux brins à ras de la ligature 60 et l'on obtient ainsi l'embout 6 selon l'invention, représenté à la figure 3E.

Selon une autre caractéristique de l'invention, le fil 7 pour ligature est choisi dans le même matériau que celui des fibres synthétiques constituant la laize du ligament 1. Ce matériau est habituellement du polyéthylène téréphalate.

Enfin, il va de soi que le procédé selon l'invention peut-être adapté à toutes autres formes de ligament que celles décrites et les exemples que l'on vient de donner ne sont que des illustrations particulières en aucun cas limitatives des domaines d'application de l'invention.

## Revendications

1. Procédé de confection d'un ligament prothétique (1) pour le remplacement d'un ligament articulaire naturel, ayant une forme globale de cylindre et comprenant entre deux parties extrêmes (2) intra-osseuses une partie médiane (3) intra-articulaire, lequel procédé comporte une étape de roulage ou pliage sur elle-même d'une laize de fibres polymères synthétiques, suivie d'une étape de pose d'un fil de traction (5) à chacune des extrémités (4) du ligament (1), puis d'une étape de mise en place d'un embout (6) sur les extrémités (4) **caractérisé en ce que** la mise en place de l'embout (6) consiste à réaliser une ligature radiale (60) du ligament (1) muni de ses fils de traction (5) avec un fil (7) pour ligature.

2. Procédé selon la revendication précédente **caractérisé en ce que** la pose du fil de traction (5) s'effectue selon la séquence suivante:
- prendre un fil de traction (5) et former une boucle (50) sans croiser les brins autour du ligament (1) et la disposer autour du ligament, à une certaine distance d'une extrémité (4) de celui-ci ;
- introduire en opposition diamétrale de la boucle (50) les deux brins libres (51,52) du fil de traction (5) dans l'épaisseur du ligament (1) et les tirer vers l'extrémité (4) considérée, parallèlement à la direction longitudinale du ligament (1) jusqu'à l'extérieur ;
- imprimer une vrille à la boucle (50) de façon à former un huit dont la base est ancrée dans le ligament (1) et passer l'extrémité (4) du ligament (1) avec les brins libres (51,52) du fil de traction (5) dans la deuxième boucle (53), c'est-à-dire la petite partie du huit ;
- ramener les deux boucles (50,53) à la hauteur du point d'introduction des brins libres (51,52) dans le ligament (1) ;
- serrer en tirant sur les brins (51,52) du fil de traction (5).

3. Procédé selon la revendication précédente **caractérisé en ce qu'**après la pose du fil de traction (5) on exécute au moins un point d'arrêt sur la section du ligament (1) dans laquelle passent les deux brins (51,52) du fil de traction (5).

4. Procédé selon l'une quelconque des revendications précédentes **caractérisé en ce que** la ligature (60) est réalisée selon la séquence suivante:
- avec un fil (7) pour ligature, former une boucle (70) allongée et la disposer sur le ligament (1) qui est posé à plat, les deux brins du fil (7) étant parallèles au ligament (1) et débouchant à l'extrémité (4) du ligament (1) sur laquelle on met en place l'embout (6),
- maintenir fermement la boucle (70) contre le ligament (1),
- prendre l'un des brins, qui devient le brin courant (71), et l'enrouler simultanément autour du ligament (1), autour du brin dormant (72) et autour de la boucle (70), en partant de l'extrémité (4) du ligament et en remontant en direction de la partie médiane (3) intra-articulaire, en formant des spires bien jointives,
- passer le brin courant (71) dans la boucle (70),
- serrer fortement en tirant sur le brin dormant (72) et le brin courant (71), tout en maintenant les spires jointives,
- couper les deux brins (71,72) à ras de la ligature.

5. Procédé selon l'une quelconques des revendications 1 ou 4 **caractérisé en ce que** le fil (7) pour ligature est choisi dans le même matériau que celui des fibres synthétiques constituant la laize.

6. Ligament (1) pour le remplacement d'un ligament articulaire naturel, ayant une forme globale de cylindre et comprenant entre deux parties extrêmes (2) intra-osseuses une partie médiane (3) intra-articulaire, muni de fils de traction (5) à ses extrémités (4) qui sont recouvertes d'un embout (6), **caractérisé en ce que** ledit embout (6) est une ligature radiale (60) du ligament (1) muni de ses fils de traction (5) réalisée avec un fil (7) pour ligature suivant le procédé selon la revendication 1.

7. Ligament (1) selon la revendication précédente, **caractérisé en ce que** ladite ligature (60) est obtenue en suivant le procédé selon la revendication 4.

## Patentansprüche

1. Verfahren zum Anfertigen einer Bandprothese (1), um ein natürliches Gelenkband zu ersetzen, die eine zylindrische Gesamtform aufweist und zwischen zwei knocheninneren Endteilen (2) einen gelenkinneren Mittelteil (3) umfasst, wobei das Verfahren einen Schritt des Rollstanzens oder Umbiegens einer Bahn aus synthetischen Polymerfasern umfasst, gefolgt von einem Schritt des Anbringens eines Zugdrahtes (5) an jedem der Enden (4) des Bandes (1), dann von einem Schritt des Aufsetzens eines Endstücks (6) auf die Enden (4), **dadurch gekennzeichnet, dass** das Aufsetzen des Endstücks (6) darin besteht, eine radiale Ligatur (60) des mit seinen Zugdrähten (5) versehenen Bandes (1) mit einem Ligaturdraht (7) zu erstellen.

2. Verfahren nach dem vorhergehenden Anspruch, **dadurch gekennzeichnet, dass** das Anbringen des Zugdrahtes (5) gemäß dem folgenden Ablauf erfolgt:
- Ergreifen eines Zugdrahtes (5) und Bilden einer Schlinge (50), ohne die Fäden um das Band (1) herum zu kreuzen, und Anordnen der Schlinge um das Band herum in einem gewissen Abstand von einem Ende (4) desselben;
- der Schlinge (50) diametrisch gegenüberliegendes Einführen der beiden freien Fäden (51, 52) des Zugdrahtes (5) in die Dicke des Bandes (1) und Ziehen derselben in Richtung auf das betreffende Ende (4) parallel zur Längsrichtung des Bandes (1) bis nach außen;
- Ausüben eines Dralls auf die Schlinge (50), um eine Acht zu bilden, deren Basis in dem Band (1) verankert ist, und Führen des Endes (4) des Bandes (1) mit den freien Fäden (51, 52) des Zugdrahtes (5) in die zweite Schlinge (53), d.h. den kleinen Teil der Acht;
- Zurückführen der beiden Schlingen (50, 53) am Einführungspunkt der freien Fäden (51, 52) in das Band (1) ;
- Festziehen durch Ziehen an den Fäden (51, 52) des Zugdrahtes (5).

3. Verfahren nach dem vorhergehenden Anspruch, **dadurch gekennzeichnet, dass** nach dem Anbringen des Zugdrahtes (5) mindestens ein Haltepunkt auf dem Abschnitt des Bandes (1) ausgeführt wird, in den die beiden Fäden (51, 52) des Zugdrahtes (5) gehen.

4. Verfahren nach einem der vorhergehenden Ansprüche, **dadurch gekennzeichnet, dass** die Ligatur (60) gemäß dem folgenden Ablauf erstellt wird:
- mit einem Ligaturdraht (7) Bilden einer länglichen Schlinge (70) und Anordnen derselben auf dem Band (1), das flach ausgelegt ist, wobei die beiden Fäden des Drahtes (7) parallel zu dem Band (1) sind und am Ende (4) des Bandes (1) ausmünden, an dem das Endstück (6) aufgesetzt wird,
- Festhalten der Schlinge (70) an dem Band (1),
- Ergreifen eines der Fäden, welcher der betreffende Faden (71) wird, und gleichzeitiges Aufwickeln desselben um das Band (1), um den stillen Faden (71) und um die Schlinge (70), ausgehend von dem Ende (4) des Bandes und in Richtung des mittleren gelenkinneren Teils (3) ansteigend, wobei dicht aneinander liegende Wicklungen gebildet werden,
- Führen des betreffenden Fadens (71) in die Schlinge (70),
- starkes Festziehen durch Ziehen an dem stillen Faden (72) und dem betreffenden Faden (71), wobei die dicht aneinander liegenden Wicklungen beibehalten werden,
- Abschneiden der beiden Fäden (71, 72) auf Höhe des Bandes.

5. Verfahren nach einem der Ansprüche 1 oder 4, **dadurch gekennzeichnet, dass** der Ligaturdraht (7) aus dem gleichen Material gewählt wird, wie das der synthetischen Fasern, welche die Bahn bilden.

6. Band (1) zum Ersatz eines natürlichen Gelenkbandes, das eine zylindrische Gesamtform aufweist und zwischen zwei knocheninneren Endteilen (2) einen gelenkinneren Mittelteil (3) umfasst, der mit Zugdrähten (5) an seinen Enden (4) versehen ist, die mit einem Endstück (6) abgedeckt sind, **dadurch gekennzeichnet, dass** das Endstück (6) eine radiale Ligatur (60) des Bandes (1) ist, das mit seinen Zugdrähten (5) versehen ist, die mit einem Ligaturdraht (7) gemäß dem Verfahren nach Anspruch 1 erstellt wird.

7. Band (1) nach dem vorhergehenden Anspruch, **dadurch gekennzeichnet, dass** die Ligatur (60) erzielt wird, indem das Verfahren nach Anspruch 4 befolgt wird.

## Claims

1. Method for production of a prosthetic ligament (1) for the replacement of a natural joint ligament, having a global cylindrical form and comprising a medial intra-articular section (3) between two intra-osseous end parts (2), said method comprising a step of the rolling or folding on itself of a web of synthetic polymer fibres, followed by a step of installation of a traction thread (5) to each of the ends (4) of the ligament (1), then a step of placing a tip (6) on the ends (4) **characterised in that** the placing of the tip (6) comprises producing a radial ligature (60) of the ligament (1) provided with the traction threads (5) by means of a ligature thread (7).

2. Method as claimed in the preceding claim **characterised in that** the installation of the traction thread (5) is carried out according to the following sequence:
- take a traction thread (5) and form a loop (50) without crossing the strands around the ligament (1) and arrange it around the ligament, at a certain distance from the end (4) of the latter;
- introduce diametrically opposed to the loop (50) the two free strands (51, 52) of the traction thread (5) into the thickness of the ligament (1) and pull them towards the end (4) under consideration, parallel to the longitudinal direction of the ligament (1) until the exterior;
- add a spiral to the loop (50) in such a way as to form an eight of which the base is anchored in the ligament (1) and pass the end (4) of the ligament (1) with the free strands (51, 52) of the traction thread (5) into the second loop (53), i.e. the small portion of the eight;
- bring the two loops (50, 53) to the point of introduction of the free strands (51, 52) in the ligament (1);
- tighten by pulling on the strands (51, 52) of the traction thread (5).

3. Method as claimed in the preceding claim **characterised in that** after the installation of the traction thread (5) at least one stop point is made on the section of the ligament (1) wherein the two strands (51, 52) of the traction thread (5) pass.

4. Method as claimed in any preceding claim **characterised in that** the ligature (60) is carried out according to the following sequence:
- by means of a ligature thread (7), form an extended loop (70) and arrange it on the ligament (1) which is installed flat, the two strands of the thread (7) being parallel to the ligament (1) and exiting at the end (4) of the ligament (1) whereon a tip (6) is set in place,
- maintain the loop (70) firmly against the ligament (1),
- take one of the strands, which becomes the current strand (71), and roll it simultaneously around the ligament (1), around the idle strand (72) and around the loop (70), starting from the end (4) of the ligament and rising back up in the direction of the medial intra-articular section (3), by forming spring coils,
- pass the current strand (71) in the loop (70),
- tighten strongly by pulling on the idle strand (72) and the current strand (71), while still maintaining the spring coils,
- cut the two strands (71, 72) right at the ligature.

5. Method according to any of claims 1 or 4 **characterised in that** the ligature thread (7) is chosen from the same material as that of the synthetic fibres comprising the web.

6. Ligament (1) for replacing a natural joint ligament, having a global cylindrical form and comprising a medial intra-articular section (3) between two intra-osseous end parts (2), provided with traction threads (5) at its ends (4) which are covered with a tip (6), **characterised in that** said tip (6) is a radial ligature (60) of the ligament (1) provided with its traction threads (5) carried out with a ligature thread (7) according to the method according to claim 1.

7. Ligament (1) as claimed in the preceding claim, **characterised in that** said ligature (60) is obtained according to the method according to claim 4.
